(19) Europäisches Patentamt
European Patent Office
Office européen des brevets

(11) EP 1 781 278 B2

(12) # NEW EUROPEAN PATENT SPECIFICATION
After opposition procedure

(45) Date of publication and mention of the opposition decision:
**16.10.2019 Bulletin 2019/42**

(45) Mention of the grant of the patent:
**10.08.2016 Bulletin 2016/32**

(21) Application number: **05767809.6**

(22) Date of filing: **30.06.2005**

(51) Int Cl.:
***A61K 31/19*** (2006.01) ***A61K 31/202*** (2006.01)
***A61K 9/00*** (2006.01) ***A61P 27/02*** (2006.01)

(86) International application number:
**PCT/US2005/023113**

(87) International publication number:
**WO 2006/007510 (19.01.2006 Gazette 2006/03)**

(54) **COMPOSITIONS AND METHODS FOR TREATING EYE DISORDERS AND CONDITIONS**

ZUSAMMENSETZUNGEN UND VERFAHREN ZUR BEHANDLUNG VON AUGENERKRANKUNGEN UND ZUSTÄNDEN

COMPOSITIONS ET PROCÉDÉS SERVANT À TRAITER DES TROUBLES ET AFFECTIONS DE L'OEIL

(84) Designated Contracting States:
**AT BE BG CH CY CZ DE DK EE ES FI FR GB GR HU IE IS IT LI LT LU MC NL PL PT RO SE SI SK TR**

(30) Priority: **01.07.2004 US 584514 P**

(43) Date of publication of application:
**09.05.2007 Bulletin 2007/19**

(73) Proprietors:
- **Schepens Eye Research**
  **Boston, MA 02114 (US)**
- **Johnson and Johnson Vision Care, Inc.**
  **Jacksonville, FL 32256 (US)**

(72) Inventors:
- **DANA, Reza**
  **Cambridge, MA 02139 (US)**
- **SCHAUMBERG, Debra**
  **Cambridge, MA 02139 (US)**
- **MOLOCK, Frank**
  **Orange Park, FL 32003 (US)**
- **COPPER, Lenora**
  **Jacksonville, FL 32223 (US)**
- **MAHADEVAN, Shiv**
  **Orange Park, FL 32003 (US)**
- **LORENZ, Kathy, Osborn**
  **Columbus, OH 43220 (US)**
- **RASHID, Saadia**
  **Boston, MA 02114 (US)**

(74) Representative: **Kirsch, Susan Edith et al**
**Carpmaels & Ransford LLP**
**One Southampton Row**
**London WC1B 5HA (GB)**

(56) References cited:
**WO-A-2004/006801** **WO-A2-01/89474**
**GB-A- 2 204 239** **US-A- 3 991 759**
**US-A- 4 911 944** **US-A1- 2002 099 100**
**US-A1- 2004 076 691**

EP 1 781 278 B2

## Description

### CROSS-REFERENCE TO RELATED APPLICATIONS

This application claims priority to U.S. Provisional Application Serial No. 60/584,514 filed July 1, 2004.

### FIELD OF THE INVENTION

The present invention relates to the treatment of eye conditions and disorders by administering compositions comprising fatty acids.

### BACKGROUND OF THE INVENTION

Dry eye syndrome (DES) is a prevalent ocular condition in the US and a frequent reason for seeking eye care. Ocular discomfort is a common patient complaint. In addition, DES -may lead to decreased functional visual acuity, problems reading, using a computer, driving at night, and carrying out professional work.

Despite progress in determining the etiology and pathogenesis of DES, current knowledge remains inadequate and the most common therapy for DES, artificial tears, provides only temporary and incomplete symptomatic relief. Therefore, identification of new forms of treatment for DES is needed.

Certain compounds known as "essential" fatty acids, are not produced by the human body, and are introduced into the system via dietary intake.. Essential fatty acids are used by the body to produce a wide array of lipid-based metabolites that are used by the body for many critical functions.

The ocular surface is normally bathed by a tear film that is composed of three layers, the most superficial of which is the lipid layer, which covers the aqueous layer of the tear film. The lipid layer of the tear film itself is composed of dozens of polar and non-polar lipid elements that interact with one another. It also contains proteins and peptides that are present in other layers of the tear film. The lipid layer has functions that relate to its physical presence on the tear film (*e.g.,* retarding evaporation) as well as to its biochemical properties (*e.g.,* providing the surface with molecules involved in the regulation of inflammation). Alterations in the lipid layer are known to be associated with a variety of ocular surface disorders, including dry eye syndrome. A majority of dry eye syndromes are associated with lipid abnormalities, including the "classic" form of aqueous or tear-deficient dry eye as is seen in Sjögren's syndrome.

Unsaturated fatty acids are characterized by the length of their hydrocarbon chain, and the number and location of their double bonds. Two groups of fatty acids are considered essential to human health and must be taken in from the diet since they cannot be synthesized from other fatty acids. These include the omega-6 fatty acids derived from linoleic acid (LA) and the omega-3 fatty acids derived from alpha linolenic acid (ALA). LA is present in high levels in the typical American diet, being found in vegetable cooking oils, beef, and dairy, and is thought to be responsible in part for promoting inflammation by negating the beneficial effects of Omega-3 FA, primarily via conversion of LA to arachidonic acid (AA). AA is also consumed directly from meat sources. It is metabolized by cyclooxygenase (COX-2) and lipoxygenase (both of which are active on the ocular surface) to form a number of powerful proinflammatory eicosanoids, including prostaglandin E2 (PGE2) and leukotriene B4 (LTB4). Gammalinolenic acid (GLA) is another omega-6 fatty acid that can be formed from LA, by action of the enzyme delta-6 desaturase, and can be further elongated to dihomogammalinolenic acid (DGLA), also an omega-6 fatty acid. In contrast to AA, GLA and DGLA are thought to lead to a reduction in inflammatory activity. However, the activity of delta-6 desaturase appears to be impaired in several diseases. Although inspection of the metabolic pathways of omega-6 fatty acids would suggest that increases in GLA should lead to increased AA, studies indicate that this does not necessarily occur. This is an important observation since DGLA, which competes with AA for oxidative enzymes, is metabolized to form prostaglandin E1 (PGE1), an eicosanoid with known anti-inflammatory and immunoregulating properties. Indeed, studies of oral GLA supplementation have shown increased production of PGE1 and reduced production of the inflammatory eicosanoids, including PGE2 and LTB4. DGLA can also modulate immune responses in a prostaglandin independent manner by acting directly on T-lymphocytes, which are thought to play a significant role in the pathology of dry eye syndrome.

The antiinflammatory effect of GLA could be enhanced in a setting in which there is an abundant supply of omega-3 fatty acids, as these fatty acids also compete with AA as enzyme substrates. Ingestion of omega-3 fatty acids reportedly results in decreased levels of membrane AA and a consequent decrease in the production of proinflammatory eicosanoids. This is paralleled by an increased production of eicosapentaenoic acid (EPA)-derived eicosanoids including the 3-series prostaglandins and thromboxanes (TXs) and the 5-series leukotrienes (LTs), which are substantially less inflammatory. The omega-3 fatty acids EPA and docosahexaenoic acid (DHA) are obtained by humans mainly through consumption of oily fish, but they may also be synthesized in the body via conversion of ALA consumed in certain seeds (e.g., flax, rape, chia, perilla and black currant) or leaves (e.g., purslane). EPA and DHA also competitively inhibit AA oxygenation by cyclooxygenase, and EPA can act as a substrate for lipoxygenase, thus further reducing the production

of the potent inflammatory AA-derived eicosanoids. Omega-3 fatty acids have also been shown to decrease the expression of adhesion molecules and the production of the proinflammatory cytokines interleukin 1 beta (IL-1 beta), interleukin 6 (IL-6)13 and tumor necrosis factor alpha (TNF-alpha). These molecules have been implicated in the pathogenesis of dry eye syndrome.

US2004/0076691 relates to compositions containing antioxidant and/or a gingkolide compound to reduce inflammation.

Oral intake of fatty acids is associated with high caloric intake and often is not tolerated well due to gastrointestinal side effects. Accordingly, there is a need for compositions and methods to treat various eye disorders and conditions, including but not limited to, dry eye syndromes and inflammation of the eye.

## SUMMARY OF THE INVENTION

The subject matter for which protection is sought is as defined in the claims.

In some embodiments, the present invention provides an ophthalmic composition for ocular administration comprising at least one omega-6 fatty acid and at least one omega-3 fatty acid, wherein the total amount of said omega-6 and said omega-3 fatty acids is from 0.01 weight % to 6 weight %; and wherein said composition is free of linoleic acid.

In some embodiments, the present invention provides the composition of the invention for use in treating dry eye by administration to an eye or ocular surface.

In some embodiments, the present invention provides the composition of the invention for use in treating adnexal inflammation by administration to an eye or ocular surface.

In some embodiments, the present invention provides a method of preparing a composition comprising an omega-6 and an omega-3 fatty acid according to the invention comprising

a) mixing a first surfactant with a saline solution; and
b) contacting at least one omega-6 acid and at least one omega-3 fatty acid with the solution of step a).

## BRIEF DESCRIPTION OF FIGURES

**Figure 1:** Tear test under specific condition: dry eye chamber ("chamber") alone, scopolamine alone, and chamber plus scopolamine.

**Figure 2:** The cornea is divided into 5 sectors as per the National Eye Institute (NEI) grading scheme and each of the five sectors is scored from 0-3 depending upon the degree of corneal fluorescein staining.

**Figure 3:** Corneal fluorescein staining scores for three groups, mice exposed to chamber alone, or scopolamine alone or combined chamber and scopolamine, for the duration of six days.

**Figure 4:** Tear measurements for four groups, groups receiving no eye drops, vehicle, or two formulations comprising omega-3 and omega-6 fatty acids. The mice were exposed to combined chamber and scopolamine continuously for six days. At 48 hours, eye drop instillation began and continued up to Day 6, at which time point, tear secretion was measured. This was the treatment arm, in which dry eye was first induced in 48 hours and then the formulations and vehicle tested.

**Figure 5:** Corneal fluorescein staining scores for three groups, group receiving no eye drops, vehicle, or formulation 1 (comprising omega-3 and omega-6 fatty acids). The mice were exposed to combined chamber and scopolamine continuously for six days. At 48 hours, eye drop instillation began and continued upto Day 6, at which time point, corneal fluorescein staining was recorded. This was the treatment arm, in which dry eye was first induced in 48 hours and then the formulations and vehicle tested.

**Figure 6:** Corneal fluorescein staining scores for three groups, group receiving no eye drops, vehicle, or formulation 2 (comprising omega-3 and omega-6 fatty acids). The mice were exposed to combined chamber and scopolamine continuously for six days. At 48 hours, eye drop instillation began and continued upto Day 6, at which time point, corneal fluorescein staining was recorded. This was the treatment arm, in which dry eye was first induced in 48 hours and then the formulations and vehicle tested.

**Figure 7:** Shows representative images of corneas stained with fluorescein, showing normalization of surface with formulations comprising omega 3 and omega 6 fatty acids.

**Figure 8:** Corneal fluorescein staining scores for three groups, group receiving no eye drops, vehicle, or formulation 2 (comprising omega-3 and omega-6 fatty acids). The mice were exposed to combined chamber and scopolamine continuously for six days. Eye drop instillation began at 0 hours. At day 6, corneal fluorescein staining was recorded. This was the prevention arm of the study, in which formulation and vehicle were tested from the start of exposure to dry eye challenge.

## DETAILED DESCRIPTION

The present invention arises, in part, out of the discovery that disorders and conditions of the eye are related to deficiencies or imbalances in essential fatty acids (*e.g.* omega-6 and/or omega-3 fatty acids).

The present invention provides an ophthalmic composition for ocular administration comprising at least one omega-6 fatty acid and at least one omega-3 fatty acid, wherein the total amount of said omega-6 and said omega-3 fatty acids is from 0.01 weight % to 6 weight %, and wherein said composition is free of linoleic acid. The composition comprises at least one omega-6 fatty acid and at least one omega-3 fatty acid. However, any number of fatty acids (e.g. essential fatty acids) may be included in the composition. Accordingly, the administration of at least one omega-6 and at least one omega-3 fatty acid would be an effective strategy to shift the ocular surface milieu toward a reduction in the production of proinflammatory mediators. In some embodiments,
the composition is substantially free of arachidonic acid.

As used herein, the term "ophthalmic composition" refers to a composition suitable for administration to the eye or ocular surface. The composition can be in any form as described herein and equivalents thereof.

As used herein, the term "about" refers to a range of ± 5% of the number that is being modified. For example, the phrase "about 10" would include both 9.5 and 10.5.

As used herein, the term "unsaturated fatty acid" refers to a fatty acid containing at least one double or triple bond. Fatty acids in this class use the Greek alphabet to identify the location of double bonds. The "alpha" carbon is the carbon closest to the carboxyl group and the "omega" carbon is the last carbon of the chain. For example, linoleic acid, and gammalinolenic acid (LA and GLA respectively) are omega-6 fatty acids because they have double bonds six carbons away from the omega carbon. Alpha-linolenic acid is an omega 3- fatty acid because it has a double bond three carbon atoms from the omega carbon. As used herein, the term "omega-3 fatty acid" refers to fatty acids that have double bonds three carbon atoms from their omega carbon atom. For example, an omega-3 fatty acid includes, but is not limited to alpha linolenic acid (ALA). Other omega-3 fatty acids include derivatives of ALA. A "derivative" of ALA is a fatty acid that is made by a chemical modification performed upon alpha linolenic acid by, for example, an enzyme or is done by organic synthesis. Examples of omega-3 fatty acids that are derivatives of ALA, include but are not limited to, eicosapentaenoic acid (EPA), docosahexaenoic acid (DHA), and the like. An "omega-3 fatty acid" can comprise one or more omega-3 fatty acids.

As used herein, the term "omega-6 fatty acid" refers to one or more fatty acids that have a double bonds 6 carbon atoms from their omega carbon atoms. For example, an omega-6 fatty acid includes, but is not limited to linoleic acid (LA). Other omega-6 fatty acids include derivatives of linoleic acid. A "derivative" of linoleic acid is a fatty acid that is made by a chemical modification performed upon linoleic acid. Examples of omega-6 fatty acids that are derivatives of linolenic acid, include but are not limited to, gammalinolenic acid (GLA), dihomogammalinolenic acid (DGLA), and the like. In some embodiments, the composition comprises at least one non-inflammatory omega-6 fatty acid. A non-inflammatory omega-6 fatty acid is an omega fatty acid that does not promote or cause inflammation. In some embodiments the inflammation is in the eye or affects the ocular surface. One of skill in the art can determine if a fatty acid causes or promotes inflammation. If the fatty acid causes or promotes inflammation, the fatty acid can be excluded from the composition.

Any method can be used to determine if a fatty acid promotes or causes inflammation. A method that determines if a fatty acid can promote or cause inflammation can be based on an increase or decrease on the infiltration of neutrophils or other cytokines in certain tissues (see, for example, Hong S et al., J Biol Chem. 2003 Apr 25;278(17):14677-87; Serhan CN, et al. J Exp Med. 2002 Oct 21;196(8):1025-37; Marcheselli VL, et al., J Biol Chem. 2003 Oct 31;278(44):43807-17; Serhan CN, et al., J Immunol. 2003 Dec 15;171(12):6856-65; Hamrah P et al, Arch Ophthalmol. 2003;121:1132-40; or Luo L et al. Invest Ophthalmol Vis Sci. 2004 Dec;45(12):4293-301.). The infiltration and activation of inflammatory cells in the cornea and conjunctiva of the eye can be assessed by performing, for example, immunohistochemical staining for markers of inflammatory cells on tissue sections in vitro, and subsequently analyzing the stained tissue specimens using scanning laser confocal microscopy. These markers include CD 3 (T-cell marker), GR-1 (neutrophil marker), CD11b (monocytic marker), F4/80 (macrophage marker) and markers of activation of inflammatory cells (MHC Class II, CD 80, CD 86, CD 40). T cells, neutrophils, monocytes and macrophages are all inflammatory cells.

The pro or anti-inflammatory effects of an agent on the eye can also be assessed by, for example, determining the protein level as well as gene expression of various pro-inflammatory cytokines such as tumor necrosis factor alpha (TNF-$\alpha$) and interleukin-1 beta (IL-1b) in the eye tissue in vitro (cornea and conjunctiva). The protein levels of the pro-inflammatory cytokines in the cornea and the conjunctiva can be assessed by performing enzyme-linked immunosorbent assay (ELISA), and the gene expression levels can be assessed by mRNA isolation, reverse-transcriptase polymerase chain reaction (PCR) and real-time PCR. An example of a fatty acid that causes or promotes inflammation is linoleic acid or arachidonic acid. If the amount of TNF-$\alpha$ and/or IL-1$\beta$ is increased the agent is said to be pro-inflammatory.

The compositions described herein are free of LA. In some embodiments, the omega-6 fatty acid is not AA

An omega-3 or an omega-6 fatty acid has a structure as indicated below.

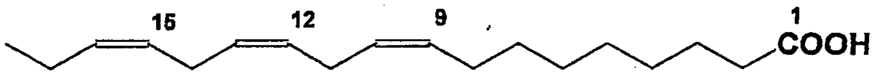

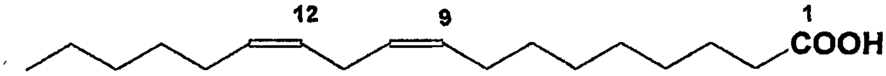

The top structure is an example of an omega-3 fatty acid. The bottom structure is an example of an omega-6 fatty acid. These structures are used for example only and other modifications are possible. Modifications can occur, for example, at the carboxyl group of the fatty acid.

As stated previously, the composition comprises at least one omega-3 fatty acid and at least one omega-6 fatty acid, wherein said composition is free of linoleic acid. In some embodiments, the composition comprises at least two omega-6 fatty acids and/or at least two omega-3 fatty acids. In some embodiments, the composition comprises at least 3, at least 4, at least 5, or least 6 omega-6 fatty acids and/or at least 3, at least 4, at least 5, or at least 6 omega 3 fatty acids. In some embodiments, the composition comprises 2, 3, 4, 5, or 6 omega-6 fatty acids. The composition can also comprise 2, 3, 4, 5, or 6 omega-3 fatty acids.

As discussed herein, the formulations disclosed herein can be prepared as ophthalmic compositions. The present invention may also be useful as a wash or irrigation solution in conscious individuals, during surgery, or to treat the dry eyes of comatose patients or those who cannot blink due to muscle or nerve damage, neuromuscular blockade or loss of the eyelids. Topical administration of a composition according to the invention comprises infusion of the preparation, composition, or topical administration from a device selected from a group consisting of a pump-catheter system, a matrix, or a sustained release device. The preparation for topical administration can comprise dispersion of the preparation in a carrier vehicle selected from a group consisting of drops of liquid, gels, ointments, and liposomes.

As used herein, the term "sustained release device" is a device that delivers an active agent or composition over a period of time. The "sustained release device" releases less than the whole amount of the active agent or composition over a period of time as opposed to releasing the entire amount of the composition or active agent all at once. A "matrix" can be made from any material that is suitable for an ophthalmic preparation or administration.

A preparation or composition according to the invention can, by way of non-limiting illustration, be applied to the eye (e.g. ocular surface) in animals and humans as a drop or within ointments, gels, or liposomes. The composition can also comprise surfactants. Further, the compositions may be infused into the tear film via a pump-catheter system. In other embodiments the compositions can be contained within continuous or other selective-release devices, for example, a membrane. In general, it is desired that the mode of application be such that the compounds enter the tear film or make contact with the surface of the eye. In some embodiments, the composition or preparation can be contained within a swab or sponge which can be applied to an ocular surface. In some embodiments, of the present invention a composition or a preparation can be contained within a liquid spray which can be applied to the ocular surface. In some embodiments, a composition or preparation can be injected directly into the lacrimal tissues or onto the eye surface.

A surgically implanted intraocular device or matrix may be a reservoir container having a diffusible wall of polyvinyl alcohol or polyvinyl acetate. Such a device or matrix containing an amount of a composition described herein may be implanted in the sclera. As another example, an amount of the composition(s) may be incorporated into a polymeric matrix having dimensions of about 2 mm x 4 mm, and made of a polymer such as polycaprolactone, poly(glycolic) acid, poly(lactic) acid, a polyanhydride, or a lipid such as sebacic acid, and may be implanted on the sclera or in the eye. This is usually accomplished with the patient receiving either a topical or local anesthetic and using a small (3-4 mm incision) made behind the cornea. The matrix, containing the composition(s), is then inserted through the incision and sutured to the sclera using 9-0 nylon.

A time-release active agent delivery system may be implanted intraocularly to result in sustained release of the omega-6 and/or omega-3 fatty acids over a period of time. The implantable formation may be in the form of a capsule of any of the polymers previously disclosed (e.g., polycaprolactone, poly(glycolic) acid, poly(lactic) acid, polyanhydride) or lipids that may be formulation as microspheres. As an illustrative example, the active agent (*e.g.,* omega-6 and/or omega-3 fatty acids) may be mixed with polyvinyl alcohol (PVA), the mixture then dried and coated with ethylene vinyl acetate, then cooled again with PVA. The active agents bound with liposomes may be applied topically, either in the form of drops or as aqueous based creams, or may be injected intraocularly. In a formulation for topical application, the active agent is slowly released over time as the liposome capsule degrades due to wear and tear from the eye surface. In a formulation for intraocular injection, the liposome capsule degrades due to cellular digestion. Both of these formulations provide advantages of a slow release active agent delivery system, providing the patient with constant dose of the active agent over time. The formulation of a sustained release is accomplished, for example, through various formulations of the vehicle-*e.g.,* coated or uncoated microsphere, coated or uncoated capsule, lipid or polymer components, unilamellar or multilamellar structure, and combinations of the above, etc.. Other variables may include the patient's

pharmacokinetic-pharmacodynamic parameters (*e.g.,* body mass, gender, plasma clearance rate, hepatic function, etc.). The formation and loading of microspheres, microcapsules, liposomes, etc. and their ocular implantation are standard techniques known by one skilled in the art, for example, the use a ganciclovir sustained-release implant to treat cytomegalovirus retinitis, disclosed in Vitreoretinal Surgical Techniques, Peyman et al., Eds. (Martin Dunitz, London 2001, chapter 45); Handbook of Pharmaceutical Controlled Release Technology, Wise, Ed. (Marcel Dekker, New York 2000).

The ophthalmic compositions of the present invention may be formed by dispersing or dissolving the selected fatty acids in a suitable carrier. Any carrier known to be ophthalmically compatible may be used to make the ophthalmic compositions of the present invention. Suitable carriers include water, saline solution, mineral oil, petroleum jelly, $C_{15-20}$ alcohols, $C_{15-20}$ amides, $C_{15-20}$ alcohols substituted with zwitterions, combinations thereof and the like. Where the selected carrier cannot dissolve the fatty acids a surfactant may be added.

In embodiments where the fatty acids are not soluble in the selected carrier it is desirable to combine the fatty acids, the carrier and surfactants, if any, to form an emulsion and in some embodiments a microemulsion. Suitable microemulsions can have droplet sizes of less than about 1 micron, less than about 0.1 micron, or less than about 0.005 microns.

The compositions can also comprise surfactants to help "solubilize" the fatty acids (*e.g.* essential and/or unessential fatty acids) such that they may be delivered to the eye of an individual.

The fatty acids are generally water insoluble and when put into a salt water (saline) solution will be even more insoluble; therefore, they need to be emulsified with surfactants that are very compatible with the long aliphatic non-polar group of the omega-3 and 6 fatty acids and a emulsification system that that will slowly release these water insoluble aliphatic acid into their surrounding environment.

The use of a surfactant may vary dramatically depending on its HLB value. The properties of a surfactant can include, but are not limited to, be surface active and reduce the surface tension to below 10 dyne/cm; be adsorbed quickly around the dispersed drops as a condensed, nonadherent film which will prevent coalescence; impart to the droplets an adequate electric potential so that mutual repulsion occurs; increase the viscosity of the emulsion; and/or be effective in a reasonable low concentration.

A pharmaceutical and/or ophthalmic acceptable surfactant can also be stable; be compatible with other ingredients; be nontoxic; possess little odor, taste or color; and/or not interfere with the stability of efficacy of the active agent

Oils also have HLB values assigned; however, this "HLB" is relative as to whether an oil-in water emulsion is to be stabilized. Surfactants, generally, should have similar HLB values to that of the respective oils in order to achieve maximum stabilization. Mineral oil has an assigned HLB between 2 and 5 depending on its number average molecular weight (Mn). Accordingly, the HLB number of the surfactant for mineral oil should be around 4 and 10.5, respectively. The desired HLB numbers can also be achieved by mixing lipophilic and hydrophilic surfactants. The overall HLB value of the mixture is calculated as the sum of the fraction times the individual HLB values.

Exemplary surfactants are Tween 80 (ethoxylated sorbitan monooleate), Glucam E-20 (ethoxylated methyl glucoside), dioleates ethoxylates based on methyl glucoside, such as but not limited to DOE 120, block copolymers comprised of a hydrophilic polyethyleneoxide (PEO) core flanked by hydrophobic polypropyleneoxide (PPO) subunits (reverse Pluronics), sorbitol ester surfactants ("SPAN" type surfactants) and combinations thereof and the like.

Table 1 shows examples of various emulsions and amounts of the surfactants that can be used when emulsifying the fatty acids. However, other amounts can also be used.

| Table 1: | Formulation 1 | | Formulation 2 | | Formulation 3 | | Formulation 4 | |
|---|---|---|---|---|---|---|---|---|
| Ratio of % of total weight Omega-3 to Omega-6 | 0.1 to 0.1 | | 0.4 to 0.1 | | 1.0 to 1.0 | | 4.0 to 1.0 | |
| | Weight (g) | % | Weight (g) | % | Weight (g) | % | Weight (g) | % |
| Glucam E-20 | 14.79 | 1.48 | 19.64 | 1.964 | 19.56 | 1.96 | 19.23 | 3.81 |
| Tween 80 | 14.71 | 1.47 | 19.8 | 1.980 | 19.61 | 1.96 | 19.43 | 3.85 |
| Saline | 968.4 | 96.85 | 955.8 | 95.557 | 941 | 94.10 | 442.03 | 87.55 |
| Omega-3 | 1.01 | 0.10 | 4.01 | 0.401 | 9.89 | 0.99 | 19.23 | 3.81 |
| Omega-6 | 0.99 | 0.10 | 0.99 | 0.099 | 9.89 | 0.99 | 4.95 | 0.98 |
| Vitamin E | 1 drop | * | 1 drop | * | 1 drop | * | 1 drop | * |

(continued)

| Table 1: | Formulation 1 | | Formulation 2 | | Formulation 3 | | Formulation 4 | |
|---|---|---|---|---|---|---|---|---|
| Ratio of % of total weight Omega-3 to Omega-6 | 0.1 to 0.1 | | 0.4 to 0.1 | | 1.0 to 1.0 | | 4.0 to 1.0 | |
| | Weight (g) | % | Weight (g) | % | Weight (g) | % | Weight (g) | % |
| Table 1: Examples of compositions used to emulsify omega-3 and omega-6 fatty acids. | | | | | | | | |

The amounts of the omega-6 and/or omega-3 fatty acid can be stated as a percentage of the total composition. The percentage of the omega-6 and/or omega-3 fatty acid can be determined by any method, but can, for example, be determined by dividing the weight of the fatty acid by the total weight of the composition. The percentage of any component of a composition can be determined in a similar manner. For example, the weight of the component is divided by the total weight of the composition to give the percentage of the component in that composition.

The total amounts of omega-6 and omega-3 fatty acid can be present in an amount equal to about 0.01, about 10 weight%, in some embodiments between about 0.01 to about 6 weight%, and in still other embodiments from about 0.05 weight% to about 5 weight %. In compositions of the invention, the total amounts of omega-6 and omega-3 fatty acid is from 0.01 weight% to 6 weight %.

The amount of omega-3 fatty acid which may be present in the ophthalmic compositions of the present invention include from about 0.01 to about 10 weight %, in some embodiments from about 0.05 weight % to about 5 weight%, based upon all the components in the ophthalmic formulation.

The amount of omega-6 fatty acid which may be present in the ophthalmic compositions of the present invention include from about 0 to about 10 weight %, in some embodiments from about 0.01 weight % to about 5 weight%, based upon all the components in the ophthalmic formulation.

In some embodiments it is desirable to provide a composition which will return the balance of omega-3 fatty acid:omega-6 fatty acid in the eye to about 1:1. To accomplish this for many people in western countries such as the United Stated and Europe, it is necessary to provide formulations which are rich in omega-3 fatty acid. Accordingly, in this embodiment it is desirable to provide ophthalmic compositions which have ratios of the omega-3 fatty acid:omega-6 fatty acid, from about 10:about 1 to no less than about 1:about1 and from about from about 5:1 to about 1:1, from about 4:1 to about 1:1, from about 3:1 to about 1:1, from about 2:1 to about 1:1, about 1:1, about 2:1, about 3:1, about 4:1, about 5:1, about 6:1, about 7:1, about 8:1, about 9:1, or about 10:1. The ratio is based on the total amount of each class of omega fatty acids.

The ophthalmic compositions of the present invention may also comprise at least one surfactant. Suitable surfactants for the ophthalmic compositions of the present invention have non-polar aliphatic tails that are similar to omega-6 and/or omega-3 fatty acids. Using surfactants that have similar aliphatic tails produces a more stable emulsion and helps to match the dispersive forces required for emulsion stability. The "like" non-polar tails of the surfactant and fatty acid also help in producing stable emulsions at higher concentrations of the material.

Surfactants are characterized according to the balance between the hydrophilic and lipophilic parts of the molecule. The hydrophilic and lipophilic balance ("HLB") is an indicator of the polarity of the molecules in a range of 1 to 40, with the most commonly used surfactants having values between 1 and 20. The HLB number increases with increasing hydrophilicity. For polymeric surfactants the HLB number may be calculated using the following formula:

$$20(\text{wt hydrophilic monomer/wt polymer surfactant})$$

$$20(1\text{-}S/A)$$

S = saponification number of the ester
A = acid number of the acid

Saponficiation number is the number of the total free and combined acids in a fat, wax or resin expressed as the number of milligrams of KOH required for the complete saponification of one gram of the substance.

Acid number is number of milligrams of KOH neutralized by the free acid in one gram of the substance.

When a saponification number cannot be obtained, the following formula may be used to determine the HLB.

$$HLB = (E + P)/5$$

Where E is the weight % of the oxyethylene
P is the weight percent of the polyhydric alcohol.

Generally, surfactants which are suitable for use in the ophthalmic compositions of the present invention have HLB numbers between about 10 and about 20, in some embodiments between about 12 and about 18 and in still other embodiments between about 14 and about 16. In some embodiments, the composition comprises about 0.5 to 20.0 weight% of surfactant, based upon all components in the composition. In some embodiments, the percentage of the surfactant is about 1 to about 15 weight %, about 1 to about 10 weight %, about 1 to about 5 weight %, and the like.

The composition may comprise more than one surfactant. The compositions of the present invention can contain other components that would facilitate the delivery of the active agent (e.g., omega-6 and/or omega-3 fatty acids). The compositions of the present invention may further comprise additional components such as antioxidants, buffer agents, tonicity adjusting agents, chelating agents, preservatives, wetting agents, thickeners, combinations thereof and the like. Suitable examples are known by those of skill in the art. In some embodiments, the composition comprises saline. In some embodiments, the composition comprises vitamin E. In some embodiments, the amount of vitamin E in the composition is equal to 1 drop (approximately 50mL).

The compositions also have other properties that can effect the properties of solubilization and active agent delivery and that, accordingly, can be modified. Examples of the types of properties that can be modified are shown in Table 2.

Table 2: Properties of formulations 1-4 shown in Table 1.

| Table 2: | pH | Conductivity | Osmolarity |
|---|---|---|---|
| Formulation 1 | 7.19 | 13.83 | 466 |
| Formulation 2 | 6.95 | 13.43 | 456 |
| Formulation 3 | 6.12 | 11.98 | 483 |
| Formulation 4 | 5.54 | 10.46 | 518 |
| Endura | | | 290 |
| Systane | | | 269 |

In some embodiments, the pH of the composition is about 5 to about 8, about 5.5 to about 7.5, about 6 to about 7.5 about 6.5 to about 7.5, about 6.9 to about 7.3, about 6.95 to about 7.2, about 7.2, about 6.95, about 6.12, about 5.54. In some embodiments, the conductivity of a composition can be about 10 to about 14, about 10 to about 12, about 10 to about 14, about 13 to about 14, about 13 to about 13.5, about 13.5 to about 14, about 12 to about 13, about 10.5, about 11, about 13.5, about 13.75, about 14, and the like. In some embodiments, the pH of the composition is 5 to 8, 5.5 to 7.5, 6 to 7.5 6.5 to 7.5, 6.9 to 7.3, 6.95 to 7.2, 7.2, 6.95, 6.12, 5.54. In some embodiments, the conductivity of a composition can be 10 to 14, 10 to 12, 10 to 14, 13 to 14, 13 to 13.5, 13.5 to 14, 12 to 13, 10.5, 11, 13.5, 13.75, 14, and the like

In some embodiments, the osmolarity of the composition is about 100 to about 600, about 150 to about 500, about 200 to about 300. In some embodiments, the osmolarity of the composition is 400 to 600, 400 to 550, 450 to 525, 100, 200, 300, 400, 500, 150, 250, 300, 350, and the like. In some embodiments, the osmolarity is less than 550, 525, 500, or 450. In some embodiments, the osmolarity is greater than 400, 425, 450, 475, 500, or 525.

In some embodiments, the invention is directed to the topical application of a composition according to the claims comprising a combination of at least one of the omega-6 fatty acids (*e.g.,* GLA and DGLA) and at least one omega-3 fatty acids (*e.g.,* including ALA, EPA and/or DHA) as an effective therapeutic strategy to decrease ocular surface inflammation. As
discussed herein the inflammation of the ocular surface can be seen in, for example, in dry eye syndrome, meibomian gland dysfunction, blepharitis, atopic keratoconjunctivitis and a wide range of other conditions. The present invention encompasses therapeutic fatty acid compounds in a therapeutically effective amount and can be dispersed in a pharmaceutically acceptable carrier vehicle suitable for ocular administration, such as, but not limited to hyaluronic acid or other methylcellulose based vehicles. A controlled release formulation is also contemplated. For example, the compositions of the invention can be administered in a sustained release formulation using a biodegradable biocompatible

polymer, or by on-site delivery using micelles, gels or liposomes. Orally, the fatty acids comprised in the composition of the invention appear to be well-tolerated in a dosage of up to several grams per day. Optimal dosage and modes of administration for topical use of the composition of the invention can readily be determined by conventional protocols.

In some embodiments, the omega-6 fatty acid compound is DGLA and the omega-3 fatty acid compounds are EPA and DHA. In some embodiments, EPA and ALA can be used as the omega-3 fatty acid combination. In some embodiments, the composition comprises either GLA or DGLA, or both, and either EPA or ALA, or both.

Additionally, the present invention includes the use of compositions in methods of treating eye conditions and disorders comprising administering to an ocular surface a composition as described herein, in particular dry eye and adnexal inflammation. The present disclosure also provides methods of increasing the age at which individuals can wear a contact lens comprising administering composition to the ocular surface. In some embodiments, the administration is a topical administration. The compositions of the present invention can also be used to treat a condition such as, but not limited to, adnexal inflammation.

The term "eye condition and disorder" includes dry eye syndromes. According to the National Eye Institute Workshop on Clinical Trials in Dry Eyes, Dry Eye Syndrome (DES) is defined as a disorder of tear film, resulting from tear deficiency and/or excessive tear evaporation, causing damage to the ocular (eye) surface and causing symptoms of ocular discomfort. The National Eye Institute Industry Workshop also produced a classification that essentially separates dry eye syndrome into two major types: tear-deficient forms (including Sjögren's syndrome and non-Sjögren's tear deficient) and evaporative forms. The tear film normally covers the front part of the eye, namely the cornea and the conjunctiva. The tear film is constantly exposed to multiple environmental factors, including variable temperature, airflow, and humidity, which may stimulate or retard its evaporation. In particular, a low humidity setting in the presence of a significant airflow increases the tear evaporation rate, as is frequently reported by subjects in desiccating environments. Indeed, even people with a normal tear secretion rate may experience dry eye symptoms while exposed to dry environments, such as in airplanes and dry workplaces. Interestingly, it has been demonstrated that ocular surface tests results for dry eye, such as the Schirmer test and tear break-up time, are decreased in subjects who live in dry climates. The present invention provides compositions for use in the treatment of such conditions as well as other conditions that are considered to be a part of dry eye since regardless of cause, all dry eye shares the "common denominator" of ocular surface dryness and epithelial damage, endpoints measured as part of the data contained herein.

An "eye condition and disorder" can also refer to, but are not limited to: keratoconjunctivitis sicca (KCS), age-related dry eye, Stevens-Johnson syndrome, Sjogren's syndrome, ocular cicatrical pemphigoid, blepharitis, corneal injury, infection, Riley-Day syndrome, congenital alacrima, nutritional disorders or deficiencies (including vitamin), pharmacologic side effects, eye stress, glandular and tissue destruction, environmental exposure (*e.g.* smog, smoke, excessively dry air, airborne particulates), autoimmune and other immunodeficient disorders, and comatose patients rendered unable to blink. Dry eye can also be defined as a condition with a decrease or change in quality of tears irrespective of the presence or absence of corneal and conjunctival lesions. It includes dry eye conditions found in the patients of hypolacrimation, alacrima, xerophthalmia, and diabetes, HIV/AIDS etc.; post-cataract surgery dry eye; allergic conjunctivitis-associated dry eye; and age-related dry-eye syndrome. Dry eye can also include the conditions found in hypolacrimation patients induced by long time visual display terminal (VDT) operations, room dryness due to air-conditioning and the like.

The present invention may also be useful as a wash or irrigation solution in conscious individuals, during surgery or to maintain comatose patients or those who cannot blink due to muscle or nerve damage, neuromuscular blockade or loss of the eyelids.

The present invention can also be administered to an individual that has been identified in need thereof of a composition described herein. The individual can be in need thereof, if the individual has been identified as suffering or having the condition of dry eye syndrome. One of skill in the art would know how to identify the patient in need of a treatment for dry eye syndrome.

In some embodiments, the present invention can also be used in methods of treating inflammation of the eye.

In some embodiments, the present invention provides methods of normalizing the ratio of omega 3 to omega 6 fatty acids in the eye. As discussed herein, the ratio of omega 3 to omega 6 fatty acids can be determined by the diet of an individual. In certain populations, the amount of omega 3 fatty acids is decreased, which can have deleterious effects such as, but not limited to dry eye syndrome or condition. Accordingly, methods comprising administering a composition according to the claims to the ocular surface or eye can be used to normalize the ratio of omega 3 fatty acids to omega 6 fatty acids. The ratio can be normalized to, for example, but not limited to about 1:1, about 2:1, about 3:1, about 4:1, about 5:1, about 1:1 to about 10:1, about 5:1 to about 10:1, or about 1:1 to about 5:1.

The compositions of the present invention can be administered to the eye as a topical preparation. In some embodiments a topical preparation comprising compositions described herein is made by combining said composition ( containing an omega-6 and omega-3 fatty acid) with an appropriate preservative. The preparation typically can also contain a physiologically compatible vehicle, as those skilled in the art can select using conventional criteria. The vehicles can be selected from the known ophthalmic vehicles which include, but are not limited to, water polyethers such as

polyethylene glycol, polyvinyls such as polyvinyl alcohol and povidone, cellulose derivative such as methylcellulose and hydroxypropyl methylcellulose, petroleum derivatives such as mineral oil, white petrolatum, animal fats such as lanolin, vegetable fats such as peanut oil, polymers of acrylic acid such as carboxypolymethylene gel, polysaccharides such as dextrans, glycosaminoglycans such as sodium hyaluronate and salts such as sodium chloride, potassium chloride, and combinations thereof. In some embodiments, the vehicle is a non-toxic ophthalmic preparation which has the following composition: about 22.0 to 43.0 millimoles of potassium per liter; about 29.0 to 50.0 millimoles of bicarbonate per liter; about 130.0 to 140.0 millimoles of sodium per liter; and about 118.0 to 136.5 millimoles of chloride per liter

In some embodiments, although it is generally desirable for the preparations to be isotonic, the final osmolarity or tonicity of the solution can vary. In some embodiments the preparation or composition can be diluted to hypotonic concentrations when this is therapeutically desirable. In some embodiments, the preparation or composition can also be concentrated to hypertonic concentrations when therapeutically desirable.

A "therapeutically effective amount" or an "effective amount" of a composition is any amount that is sufficient to provide the outcome desired. One of skill in the art would readily be able to determine what is a therapeutically effective amount or an effective amount. A therapeutically effective amount can refer to an amount of a composition effective to prevent, alleviate or ameliorate symptoms of a disease, a disorder, or a condition in an individual.

In some embodiments, the compositions according to the present invention can be ophthalmic preparations. Ophthalmic compositions or preparations are formulated according to the mode of administration to be used. Generally, additives for isotonicity can include sodium chloride, dextrose, mannitol, sorbitol, and lactose. In some embodiments, isotonic solutions such as phosphate buffered saline are used. Stabilizers include gelatin and albumin. In some embodiments, a vasoconstriction agent is added to the formulation. The ophthalmic preparations according to the present invention can be sterile and pyrogen free. Pharmaceutical (*e.g.* ophthalmic) compositions according to the invention include delivery components in combination with the compositions described herein which further comprise a ophthalmically acceptable carrier or vehicles, such as, for example, saline solutions. Any medium can be used which allows for successful delivery of compositions described herein. One skilled in the art would readily comprehend the multitude of pharmaceutically (*e.g.,* ophthalmically) acceptable media that can be used in the present invention. Suitable ophthalmic carriers are described in *Remington's Pharmaceutical Sciences, A. Osol,* a standard reference text in this field.

The compositions and/or ophthalmic compositions according to the present invention can be administered as a single dose or in multiple doses. The ophthalmic compositions of the present invention can be administered either as individual therapeutic agents or in combination with other therapeutic agents. The treatments of the present invention may be combined with conventional therapies, which can be administered sequentially or simultaneously.

Dosage varies depending upon known factors such as the pharmacodynamic characteristics of the particular agent, and its mode and route of administration; age; health and weight of the recipient; nature and extent of symptoms; kind of concurrent treatment; frequency of treatment; and the effect desired. Formulation of therapeutic compositions and their subsequent administration is within the skill of those in the art. Usually, the dosage can be about 1 to 3000 milligrams per 50 kilograms of body weight; 10 to 1000 milligrams per 50 kilograms of body weight; 25 to 800 milligrams per 50 kilograms of body weight. Dosage can also be formulated as a concentration of the active ingredient (*e.g.* omega-6 and/or omega-3 fatty acids) in a solution, such as an eye drop.

The present invention also provides methods of preparing or a process of making a composition (*e.g.* pharmaceutical) comprising an omega-6 and an omega-3 fatty acid according to the invention. The method comprises mixing (*e.g.,* stirring) at least one or a first surfactant with a saline solution. The saline solution can be any solution that is appropriate

for the composition, for example, but not limited to a borate buffered saline solution. In some embodiments a second surfactant is contacted (*e.g.* mixed) with a solution comprising a first surfactant and the saline solution. In some embodiments, the process occurs at room temperature. In some embodiments, the fatty acid (*e.g.,* omega-6 and/or omega-3) is added over a certain time period. In some embodiments, the time period is about 30 minutes, about 1 hour, about 2 hours, about 3 hours, about 4 hours, about 5 hours. When a substance (solution or solid) is added over a certain time period it is added in about equal amounts over that time period. It can be added at any interval (*e.g.,* 5 minutes, 10 minutes, 15 minutes, 20 minutes, 30 minutes, 40, minutes, 60 minutes, and the like).

The mixing speed can also be modified such that at first the mixing is done slowly and then the RPM of the mixing apparatus is increased. Any mixing apparatus can be used. In some embodiments, the mixing apparatus is a magnetic stir bar (or paddle) in a solution on top of a magnetic stirrer. Other mixing apparatuses include mechanical stirrers equipped with stirring shafts In some embodiments, the mixing is begun at 5 RPM and is increased at a rate of 5 rpm every 2 minutes. In some embodiments, the maximum RPM of the mixing step(s) is 5, 10, 15, 20, 25, 30, 35, 40, 45, 50, 60, 70, 80, 90, 100, 150, 200, less than 200, less than 150, less than 100, less than 50, less than 25. In some embodiments, the rpm of the mixing step(s) is from about 50 to about 75, 50 to 75, about 40 to about 100, about 50 to about 100, 50 to 100, 40 to 100, and the like. In some embodiments the rpm of the mixing apparatus is increased to 100 rpm. During the mixing steps the mixing can be stopped for a period of time. In some embodiments, the stirring is stopped for about 5, about 10, about 15, about 20, about 30, about 40, about 50, about 60, about 70, about 80, about 90, less

than 30, more than 30, less than 60, more than 60, 5, 10, 15, 20, 30, 40, 50, 60, 70, 80, 90 minutes, and the like.

In some embodiments, vitamin E is added to a solution comprising a first surfactant and a fatty acid.

The stirring (*e.g.* mixing) steps described herein can be for any time period that thoroughly mixes the solutions to a desired state. A desired state or condition can be determined by one of skill in the art. In some embodiments, the solutions are stirred for about 5 minutes, about 10 minutes, about 15 minutes, about 20 minutes, about 30 minutes, about 40 minutes, about 50 minutes, about 60 minutes, about 1 to about 2 hours, about 1 to about 3 hours, about 1 to about 4 hours, about 1 to about 5 hours, about 3 to about 4 hours, about 2 to about 4 hours, about 6 hours, more than 2 hours, more than 3 hours, more than 4 hours, overnight (*e.g.* about 8 to about 12 hours), and the like.

In some embodiments, a first surfactant is added to a saline solution to make a surfactant-saline solution. In some embodiments, a second surfactant is added to such a solution to make a system comprising a two surfactant-saline solution. In some embodiments at least one omega-6 fatty acid and/or at least one omega-3 fatty acid is added to the two-surfactant-saline solution to make a solution comprising at least one fatty acid and a two surfactant-saline solution. In some embodiments, about 1 drop, about 2 drops, about 3 drops, about 4 drops, about 5 drops, at least 5 drops, at least 10 drops, at least 1 drop, at least 2 drops, at least 3 drops, at least 4 drops, at least 20 drops, 1 drop, 2 drops, 3 drops, 4 drops, 5 drops, 6 drops, 7 drops, 8 drops, 9 drops, or 10 drops of Vitamin E is added to the fatty acid-surfactant-saline solution. In some embodiments, other antioxidants are added to prevent oxidation of the fatty acids. Other antioxidants can be used in the place of or in addition to the vitamin E drop(s) such that the oxidation of the fatty acid chains is prevented or delayed.

The invention is now described with reference to the following examples. These examples are provided for the purpose of illustration only and the invention should in no way be construed as being limited to these examples but rather should be construed to encompass any and all variations which become evident as a result of the teaching provided herein.

**Examples**

**Example 1: Dry Eye Animal Model:**

Normal healthy mice can be induced to have dry eye by continuously exposing them to dry environment in a controlled environmental chamber (CEC), described below. Mice in CEC were continuously exposed throughout the duration of the experiments, to low relative humidity of less than 30% (mean and standard deviation 19% $\pm$ 4%), high airflow (151it/ minute) and constant temperature (21-23 Celsius). Mice in normal cages were exposed to relative humidity over 70% (mean and SD 78% $\pm$ 5%), no airflow and same temperature. In addition, the mice placed in CEC were also treated with scopolamine, a active agent that causes pharmacological inhibition of tear secretion. The combination of CEC and scopolamine produces severe dry eye.

Sustained-release transdermal scopolamine patches (scop patch) were obtained from Novartis (Summit, NJ). One-fourth of the patch is applied to the depilated mid-tail of mice every 48 hours.

**Controlled Environmental Chamber:**

The CEC was created allowing regulation of air flow and humidity, and control of temperature. The chamber consisted of a cage (Lab Products Inc., Seaford, DE) modified in order to allow the use of desiccants. The usable floor area of our modified cage was 725 $cm^2$. The roof of the cage was sealed with isolating material in order to make the chamber independent from the humidity of the room where it is placed. A hole in the roof allowed the air to move outside the CEC. A stainless-steel barrier system had been placed into the chamber in order to place desiccants without the risk of any contact with the mice. For desiccants, indicating silica gel, packed in cartridges of 114 mm diameter (Cole-Parmer Instrument Company, Vernon Hills, IL), and indicating Drierite (anhydrous $CaSO_4$; W.A. Hammond Drierite Co., Xenia, OH), both of which are commonly used to remove moisture from the environment, and are non-toxic for humans and animals were used. The CEC was connected to an air line and a temperature and humidity recorder. A small low-noise (38 dB) oil-less linear pump (38 liter per minute open flow, 26 watts; Gast Manufacturing Inc., Benton Harbor, MI) placed 1 m from the chamber was used as the source of air. The flow was regulated by a flowmeter (0-50 l/min, accuracy $\pm$ 5%) with a valve placed on the air line. The air was pumped into the chamber through 4 tips (1 mm diameter) placed in two opposite walls, in order to create a laminar flux of air, and avoiding turbulences. The height of the tips (3.5 and 4.5 cm on one side, 3 and 4 cm on the other) was chosen to correspond to the height of the mice's eyes. The humidity of the air pumped in the chamber can be regulated by a valve which can direct the air into a desiccant system made of a water separator (SMC Corporation, Tokyo, Japan), and air drying columns containing Drierite. In the CEC, temperature (range 5-45 °C, accuracy $\pm$ 1°C), and humidity (0 - 100%, accuracy 2%) were constantly monitored by a probe, and recorded on circular charts by a temperature humidity recorder (Supco, Allenwood, NJ).

**Ocular Surface Tests for Signs of Dry Eye:**

Signs of dry eye were assessed by performing: a) cotton thread test to measure aqueous tear production, which is generally decreased in patients with DES; and b) corneal fluorescein staining which is a marker of corneal surface damage, and is generally increased in patients with DES.

**Cotton Thread Test:**

Tear production was measured with cotton thread test, impregnated with phenol red (Zone-Quick, Lacrimedics, Eastsound, WA). The validity of this test in mice has previously been described. Under a magnifying fluorescent lamp, the thread was held with jeweler forceps and placed in the lateral cantus of the conjunctival fornix of the right eye for 60 seconds. The tear distance in mm is read under a microscope (Zeiss S4, West Germany) using the scale of an hemacytometer. Tear secretion was measured in three groups of mice (Figure 1). In mice treated in the CEC alone for 6 days tear secretion decreased significantly as compared to the baseline (Day 0) at Day 3 and by Day 6 (Figure 1). Second group of mice were treated with Scop patch alone for 6 days (Figure 1) and the third group with both CEC and Scop patch for 6 days (Figure 1). In both the second and third groups of mice, tear secretion was markedly decreased at both Day 3 and Day 6 as compared to the baseline, the effect was more profound than the chamber alone and was sustained throughout the duration of the experiment.

**Corneal Fluorescein Staining:**

Corneal fluorescein staining was performed by applying 1.0 μl of 5% fluorescein by a micropipette into the inferior conjunctival sac of the eye. The cornea was examined with a slit lamp biomicroscope using cobalt blue light 3 minutes after the fluorescein instillation. Punctuate staining was recorded in a masked fashion using a standardized National Eye Institute (NEI) grading system of 0-3 for each of the five areas in which the corneal surface has been divided (Figure 2).

As shown in Figure 3, the chamber alone or scopolamine alone produced significant increase in corneal fluorescein staining as compared to the controls both at Day 3 and Day 6. However, the staining was most markedly increased with the combined effect of scopolamine and the chamber at each time point.

This model recreated clinical dry eye in normal mice

**Example 2: Formulations**

Formulation 3 comprises 1% EPA+DHA and 1% GLA and formulation 1 comprises 0.1 % of the fatty acids. Formulations 2 and 4 comprise a 4:1 ratio of omega-3 to omega-6 ratio. The formulations were constituted in mineral oil as the vehicle and Vitamin E was added as an anti-oxidation agent. The fatty acids were stored in dark to further decrease the risk of oxidation. The formulations are summarized in Table 3.

Table 3: Fatty Acid Formulations. Molar ratios of EPA:DHA in all formulations is 1:1. Molecular weight of EPA: 302.5; Molecular weight of DHA: 328.6;Molecular weight of GLA: 278.4

| | Omega-3 (EPA+DHA)<br>**a) concentration (wt/vol %)**<br>b) Quantity (wt/vol)<br>c) Molarity<br>d) EPA/DHA quantity wt/vol | Omega-6 (GLA)<br>**a) concentration (wt/vol %)**<br>b)Quantity (wt/vol)<br>c) Molarity | Concentration Ratio of EPA+DHA:GLA |
|---|---|---|---|
| Formulation 1 (Low concentration) | a) **0.1%**<br>b) 1mg/ml<br>c) 3.17mM<br>d) **0.5mg/ml EPA: 0.5mg/ml DHA.** | a) **0.1%**<br>b) **1mg/ml GLA**<br>c) 3.6mM | 1:1 |

(continued)

| | Omega-3 (EPA+DHA)<br>**a) concentration (wt/vol %)**<br>b) Quantity (wt/vol)<br>c) Molarity<br>d) EPA/DHA quantity wt/vol | Omega-6 (GLA)<br>**a) concentration (wt/vol %)**<br>b)Quantity (wt/vol)<br><br>c) Molarity | Concentration Ratio of EPA+DHA:GLA |
|---|---|---|---|
| Formulation 2 (Low concentration) | a) **0.4%**<br>b) 4mg/ml<br>c) 12.7mM<br>d) **2mg/ml EPA: 2mg/ml DHA** | a) **0.1%**<br>b) **1mg/ml GLA**<br>c) 3.6mM | 4:1 |
| Formulation 3 (High concentration) | a) **1%**<br>b) 10mg/ml<br>c) 31.7mM<br>d) **5mg/ml EPA: 5mg/ml DHA.** | a) **1%**<br>b) **10mg/ml GLA**<br>c) 35.9mM | 1:1 |
| Formulation 4 (High concentration) | a) 4%<br>b) (40mg/ml)<br>c) 126.8mM<br>d) **20mg/ml EPA: 20mg/ml DHA** | a) **1%**<br>b) **10mg/ml GLA**<br>c) 35.9mM | 4:1 |

**Example 3: Active Agent Study Design**

The study was a prospective masked trial. One eye each mouse was randomized to receive either vehicle (negative control), or one of the formulations. The active agents were administered in a masked fashion. 5μl eye drops were administered in one of the eye, twice a day, that is the interval between the doses is 12 hours.

Dry eye was induced by combined environmental and pharmacological effect (placing the mice in CEC and applying scopolamine patch for 6 days). There were two main groups of the active agent trial. Treatment group: Eye drops were instilled after exposure to CEC+Scop patch for 48 hours. Active agent administration continues from 48 hours to Day 6. Signs of dry eye were assessed at the end of Day 6, twelve hours after the last dose. Prevention group: Eye drops were instilled from Day 0 and continues up to Day 6. Signs of dry eye were assessed at the end of Day 6, twelve hours after the last dose.

**Statistical analysis**

The student *t* test for the aqueous tear production, and the Mann-Whitney test for corneal fluorescein staining were used to compare the differences between the vehicle and the formulation groups. Within group changes from baseline were analyzed by the paired *t* test (aqueous tear production) and by the Wilcoxon test (corneal fluorescein staining). All tests were two-tailed, and a *p* value less then 0.05 was considered to be statistically significant.

**Results:**

**Tear Test: Treatment with Formulation 1 and Formulation 2** Figure 4 summarizes the results of tear measurements at Day 0, Day 2 and Day 6 for four groups, group receiving no eye drops, vehicle, formulation 1 or formulation 2. At Day 0, all four groups had normal tear secretion. Forty-eight hours after exposure to CEC+Scop patch, all four groups developed signs of dry eye, that is, significant decrease in tear secretion as compared to the baseline (Day 0). At this point (48 hours), 5ul of eye drops per eye was administered every 12 hours as the mice continued to be exposed

to CEC+Scop. At Day 6, the group receiving no eye drops continued to have significant decrease in tear secretion as compared to the baseline (Day 0) and as compared to the groups receiving the vehicle or formulation 1 or 2. The groups receiving either the vehicle or the formulations had an increase in tear secretion as compared to 48 hours (dry eye prior to active agent administration) but the tear values did not reach that of baseline (Day 0). Thus in the tear test (a secondary endpoint), both formulations 1 and 2 were effective in reversing the decreased tearing in the dry eye.

**Corneal Fluorescein Staining: Treatment with Formulations 1 and 2**

Figures 5 and 6 summarize the results of corneal fluorescein scores for eyes receiving no eye drops, vehicle, formulation 1 or formulation 2. All groups were similar at day 0 (baseline). Forty-eight hours after exposure to CEC+Scop patch, all groups developed an increase in corneal fluorescein staining as compared to the baseline (day 0). Eye drop instillation began at 48 hours and continued until Day 6 as the mice in all the groups were continuously exposed to CEC+Scop patch.

Both at day 4 and day 6, the vehicle showed no significant decrease in corneal fluorescein staining as compared to the group receiving no eye drops, and as compared to day 2 (Figures 5 and 6. At days 4 and 6, the corneal fluorescein staining in vehicle group continued to be significantly higher than the baseline (Day 0).

Formulation 1 showed a significant decrease in corneal fluorescein staining both at days 4 and 6, as compared to Day 2 and also as compared to groups receiving vehicle or no eye drops (Figure 5). Furthermore, corneal fluorescein staining scores in eyes receiving formulation 1 returned to baseline (normal values) by Day 6.

Eyes receiving formulation 2 (Figure 6) showed a significant decrease in corneal fluorescein staining at day 4 as compared to the vehicle and eyes receiving no eye drops at Day 4. There was also a significant decrease within group, as compared to Day 2 (48 hours). At Day 6, the fluorescein staining scores increased slightly, however formulation 2 still showed decreased (though not statistically significant) staining scores as compared to the vehicle. Both at Day 4 and Day 6, the formulation 2 group had significantly lower staining scores as compared to eyes receiving no drops.

Figure 7 shows representative images of corneas stained with fluorescein, showing normalization of corneal surface with formulations 1 & 2.

Both formulations 1 and 2 had a profound and significant and sustained effect in suppressing corneal epithelial disease (as measured by staining). This suppressive effect in treating dry eye was significantly more potent than what the application of the vehicle alone offered.

**Corneal Fluorescein Staining: Prevention with Formulation 2** Figure 8 summarizes the results of corneal fluorescein scores for eyes receiving no eye drops, vehicle or formulation 1. In the prevention groups, eye drop instillation began at Day 0 and continued until Day 6. All mice were exposed to CEC+Scop patch throughout this time. At day 6, the vehicle group showed borderline significant decrease in fluorescein staining scores as compared to group receiving no eye drops. At day 6, formulation 2 showed significant decrease in staining scores both as compared to the vehicle and the group receiving no eye drops.

Thus, formulations 1 and 2 were shown to lead to a decrease in dry eye signs. Formulations 1 and 2 demonstrated therapeutic efficacy compared to vehicle application in regard to the primary outcome, corneal staining.

**Example 4: Preparation of Formulations**

In a typical emulsion the solubilizing material, Glucam E-20 is added to borate buffered saline (%wt of each component: NaCl 0.83; $H_3BO_3$0.89; $Na_2B_4O_7.10H_2O$ 0.23; EDTA 0.01; $H_2O$ 98.04). This solution was stirred for at least 30 minutes at room temperature after the solution had become homogeneous. The stirring started out with the stirring being increased 5 rpm for each 2 minute interval until 100 rpm is achieved. The stirring blade should be paddle like so that high shear stirring is obtained during mixing. After the solution had been stirred for at least 30 minutes at 100rpm a clear solution was achieved.

The clear solution was allowed to stand with no stirring for 10 minutes at which time the Tween 80 was added. At first the material appeared to be insoluble and wrapped around the stirring shaft, but after all the Tween had been added the stirring was resumed and the Tween 80 dissolved using the same stirring procedure that was used with the Glucam-E at room temperature.

With the solution stirring between 50 and 75 rpm the fatty acid was slowly added over a one hour period, after this period the solution can appear milky. At the end of the hour period the fatty acid was added over an hour period keeping the stirring between 50 and 75 rpm. At this time the solutions will appear to be white if higher concentrations of fatty acids are used. Low concentration solutions produced microemulsions and appear translucent. After this time the solution was stirred and one drop of Vitamin E was added and the solution continued to stir for three to four hours at the shear rate that has been obtained, e.g., 75 rpm.

While this invention has been disclosed with reference to specific embodiments, it is apparent that other embodiments and variations of this invention may be devised by others skilled in the art without departing from the scope

of the invention. The appended claims are intended to be construed to include all such embodiments and equivalent variations.

## Claims

1. An ophthalmic composition for ocular administration comprising at least one omega-6 fatty acid and at least one omega-3 fatty acid, wherein the total amount of said omega-6 and said omega-3 fatty acids is from 0.01 weight % to 6 weight %; and wherein said composition is free of linoleic acid.

2. The composition of claim 1, wherein the total amount of said omega-6 and said omega-3 fatty acid is from 0.05 weight % to 5.0 weight %.

3. The composition of claim 1 or claim 2, wherein said composition is also free of arachidonic acid.

4. The composition of claim 1 or claim 2, wherein said composition comprises a surfactant.

5. The composition of claim 4, wherein the surfactant is 1 to 15 weight %.

6. The composition of claim 4, wherein the surfactant is 1 to 10 weight %.

7. The composition of claim 4, wherein said surfactant is ethoxylated sorbitan monooleate, ethoxylated methyl glucoside, a dioleate ethoxylate based on methyl glucoside, reverse Pluronic surfactants, SPAN surfactants or mixtures thereof.

8. The composition of claim 1 or claim 2, wherein said composition comprises eye drops.

9. The composition of claim 1 or claim 2, wherein said omega-6 fatty acid compound is a noninflammatory omega-6 fatty acid.

10. The composition of claim 1 or claim 2, wherein said omega-3 fatty acid compound is alpha linolenic acid.

11. The composition of claim 1 or claim 2, wherein said omega-6 fatty acid compound is gammalinolenic acid, dihomogammalinolenic acid, or both.

12. The composition of claim 1 or claim 2, wherein said omega-3 fatty acid compound is eicosapentaenoic acid, docosahexaenoic acid, or both.

13. The composition of claim 1 or claim 2, wherein said composition is formulated for topical administration.

14. The composition of claim 1 or claim 2, wherein said composition comprises a sustained release device.

15. The composition of claim 1 or claim 2, wherein ratio of said omega 3 fatty acid to omega 6 fatty acid is 10:1 to 1:1.

16. The composition of claim 1 or claim 2, wherein ratio of said omega 3 fatty acid to omega 6 fatty acid is 5:1 to 1:1.

17. The composition of claim 1 or claim 2, wherein ratio of said omega 3 fatty acid to omega 6 fatty acid is 5:1.

18. The composition of claim 1 or claim 2, wherein ratio of said omega 3 fatty acid to omega 6 fatty acid is 1:1.

19. The composition of claim 1, wherein the concentration of omega-3 fatty acid is 0.05 weight % to 5 weight %.

20. The composition of claim 1 or claim 2, wherein the composition is sterile.

21. The composition of any one of claims 1 to 20 for use in treating dry eye by administration to an eye or ocular surface.

22. The composition of any one of claims 1 to 20 for use in treating adnexal inflammation by administration to an eye or ocular surface.

**23.** A method of preparing a composition comprising an omega-6 and an omega-3 fatty acid as claimed in any one of claims 1 to 20 and 27, said method comprising

a) mixing a first surfactant with a saline solution; and
b) contacting at least one omega-6 acid and at least one omega-3 fatty acid with the solution of step a).

**24.** The method of claim 23, wherein said mixing and/or said contacting is done at room temperature.

**25.** The method of claim 23, wherein said at least omega-6 fatty acid and at least one omega-3 fatty acid is contacted with a solution of step a) over an hour.

**26.** The method of claim 23, wherein a second surfactant is mixed with a solution of step a) prior to step b).

**27.** The composition of claim 1, wherein the amount of said omega-6 fatty acid is 0.005 weight %, and wherein the amount of said omega-3 fatty acid is 0.005 weight %.

**Patentansprüche**

**1.** Ophthalmische Zusammensetzung zur okularen Verabreichung, welche zumindest eine Omega-6-Fettsäure und zumindest eine Omega-3-Fettsäure aufweist, wobei die Gesamtmenge der Omega-6- und Omega-3-Fettsäuren zwischen 0,01 Gewichts-% und 6 Gewichts-% liegt; und wobei die Zusammensetzung frei von Linolsäure ist.

**2.** Zusammensetzung nach Anspruch 1, wobei die Gesamtmenge der Omega-6- und Omega-3-Fettsäure zwischen 0,05 Gewichts-% und 5,0 Gewichts-% liegt.

**3.** Zusammensetzung nach Anspruch 1 oder Anspruch 2, wobei die Zusammensetzung außerdem frei von Arachidonsäure ist.

**4.** Zusammensetzung nach Anspruch 1 oder Anspruch 2, wobei die Zusammensetzung ein Tensid aufweist.

**5.** Zusammensetzung nach Anspruch 4, wobei das Tensid 1 bis 15 Gewichts-% ausmacht.

**6.** Zusammensetzung nach Anspruch 4, wobei das Tensid 1 bis 10 Gewichts-% ausmacht.

**7.** Zusammensetzung nach Anspruch 4, wobei das Tensid ethoxyliertes Sorbitanmonooleat, ethoxyliertes Methylglucosid, ein auf Methylglucosid basierendes Dioleat-Ethoxylat, umgekehrte Pluronic-Tenside, SPAN-Tenside oder Mischungen davon ist.

**8.** Zusammensetzung nach Anspruch 1 oder Anspruch 2, wobei die Zusammensetzung Augentropfen aufweist.

**9.** Zusammensetzung nach Anspruch 1 oder Anspruch 2, wobei die Omega-6-Fettsäureverbindung eine nicht entzündliche Omega-6-Fettsäure ist.

**10.** Zusammensetzung nach Anspruch 1 oder Anspruch 2, wobei die Omega-3-Fettsäureverbindung Alpha-Linolensäure ist.

**11.** Zusammensetzung nach Anspruch 1 oder Anspruch 2, wobei die Omega-6-Fettsäureverbindung Gammalinolensäure, Dihomogammalinolensäure oder beide von diesen ist.

**12.** Zusammensetzung nach Anspruch 1 oder Anspruch 2, wobei die Omega-3-Fettsäureverbindung Eicosapentaensäure, Docosahexaensäure oder beides ist.

**13.** Zusammensetzung nach Anspruch 1 oder Anspruch 2, wobei die Zusammensetzung zur topischen Verabreichung formuliert ist.

**14.** Zusammensetzung nach Anspruch 1 oder Anspruch 2, wobei die Zusammensetzung eine Retard-Vorrichtung aufweist.

15. Zusammensetzung nach Anspruch 1 oder Anspruch 2, wobei das Verhältnis der Omega-3-Fettsäure zu der Omega-6-Fettsäure 10:1 bis 1:1 beträgt.

16. Zusammensetzung nach Anspruch 1 oder Anspruch 2, wobei das Verhältnis der Omega-3-Fettsäure zu der Omega-6-Fettsäure 5:1 bis 1:1 beträgt.

17. Zusammensetzung nach Anspruch 1 oder Anspruch 2, wobei das Verhältnis der Omega-3-Fettsäure zu der Omega-6-Fettsäure 5:1 beträgt.

18. Zusammensetzung nach Anspruch 1 oder Anspruch 2, wobei das Verhältnis der Omega-3-Fettsäure zu der Omega-6-Fettsäure 1:1 beträgt.

19. Zusammensetzung nach Anspruch 1, wobei die Konzentration der Omega-3-Fettsäure 0,05 Gewichts-% bis 5 Gewichts-% beträgt.

20. Zusammensetzung nach Anspruch 1 oder Anspruch 2, wobei die Zusammensetzung steril ist.

21. Zusammensetzung nach einem der Ansprüche 1 bis 20 zur Verwendung bei der Behandlung von trockenen Augen durch Verabreichung an ein Auge oder eine okulare Oberfläche.

22. Zusammensetzung nach einem der Ansprüche 1 bis 20 zur Verwendung bei der Behandlung von Adnexaentzündung durch Verabreichung an ein Auge oder eine okulare Oberfläche.

23. Verfahren zum Herstellen einer Zusammensetzung, die eine Omega-6- und eine Omega-3-Fettsäure nach einem der Ansprüche 1 bis 20 und 27 aufweist, welches aufweist:

   a) Mischen eines ersten Tensids mit einer Salzlösung; und
   b) Inkontaktbringen zumindest einer Omega-6-Säure und zumindest einer Omega-3-Fettsäure mit der Lösung aus Schritt a).

24. Verfahren nach Anspruch 23, wobei das Mischen und/oder Inkontaktbringen bei Raumtemperatur erfolgt.

25. Verfahren nach Anspruch 23, wobei die zumindest Omega-6-Fettsäure und die zumindest eine Omega-3-Fettsäure mit einer Lösung aus Schritt a) über eine Stunde in Kontakt gebracht werden.

26. Verfahren nach Anspruch 23, wobei ein zweites Tensid mit einer Lösung aus Schritt a) vor Schritt b) gemischt wird.

27. Zusammensetzung nach Anspruch 1, wobei die Menge der Omega-6-Fettsäure 0,005 Gewichts-% beträgt und wobei die Menge der Omega-3-Fettsäure 0,005 Gewichts-% beträgt.

**Revendications**

1. Composition ophtalmique pour une administration oculaire, comprenant au moins un acide gras oméga-6 et au moins un acide gras oméga-3, dans laquelle la quantité totale desdits acides gras oméga-6 et oméga-3 est de 0,01 % en poids à 6 % en poids ; et dans laquelle ladite composition est exempte d’acide linoléique.

2. Composition selon la revendication 1, dans laquelle la quantité totale dudit acide gras oméga-6 et dudit acide gras oméga-3 est de 0,05 % en poids à 5,0 % en poids.

3. Composition selon l’une des revendications 1 ou 2, dans laquelle ladite composition est aussi exempte d’acide arachidonique.

4. Composition selon l’une des revendications 1 ou 2, dans laquelle ladite composition comprend un tensio-actif.

5. Composition selon la revendication 4, dans laquelle le tensio-actif représente de 1 à 15 % en poids.

6. Composition selon la revendication 4, dans laquelle le tensio-actif représente de 1 à 10 % en poids.

**7.** Composition selon la revendication 4, dans laquelle ledit tensio-actif est le monooléate de sorbitan éthoxylé, le méthyl glucoside éthoxylé, un dioléate éthoxylate à base de méthyl glucoside, les tensio-actifs Pluronic inverses, les tensio-actifs SPAN ou les mélanges de ceux-ci.

**8.** Composition selon l'une des revendications 1 ou 2, dans laquelle ladite composition comprend des gouttes ophtalmiques.

**9.** Composition selon l'une des revendications 1 ou 2, dans laquelle ledit composé acide gras oméga-6 est un acide gras oméga-6 non inflammatoire.

**10.** Composition selon l'une des revendications 1 ou 2, dans laquelle ledit composé acide gras oméga-3 est l'acide alpha linolénique.

**11.** Composition selon l'une des revendications 1 ou 2, dans laquelle ledit composé acide gras oméga-6 est l'acide gammalinolénique, l'acide dihomogammalinolénique ou les deux.

**12.** Composition selon l'une des revendications 1 ou 2, dans laquelle ledit composé acide gras oméga-3 est l'acide eicosapentaénoïque, l'acide docosahexaénoïque ou les deux.

**13.** Composition selon l'une des revendications 1 ou 2, dans laquelle ladite composition est formulée pour une administration topique.

**14.** Composition selon l'une des revendications 1 ou 2, dans laquelle ladite composition comprend un dispositif à libération entretenue.

**15.** Composition selon l'une des revendications 1 ou 2, dans laquelle le rapport dudit acide gras oméga-3 audit acide gras oméga-6 est 10:1 à 1:1.

**16.** Composition selon l'une des revendications 1 ou 2, dans laquelle le rapport dudit acide gras oméga-3 audit acide gras oméga-6 est 5:1 à 1:1.

**17.** Composition selon l'une des revendications 1 ou 2, dans laquelle le rapport dudit acide gras oméga-3 audit acide gras oméga-6 est 5:1.

**18.** Composition selon l'une des revendications 1 ou 2, dans laquelle le rapport dudit acide gras oméga-3 audit acide gras oméga-6 est 1:1.

**19.** Composition selon la revendication 1, dans laquelle la concentration de l'acide gras oméga-3 est 0,05 % en poids à 5 % en poids.

**20.** Composition selon l'une des revendications 1 ou 2, dans laquelle la composition est stérile.

**21.** Composition selon l'une quelconque des revendications 1 à 20 pour une utilisation dans le traitement de l'oeil sec par administration à un oeil ou à une surface oculaire.

**22.** Composition selon l'une quelconque des revendications 1 à 20 pour une utilisation dans le traitement d'une inflammation des annexes de l'oeil par administration à un oeil ou à une surface oculaire.

**23.** Procédé de préparation d'une composition comprenant un acide gras oméga-6 et un acide gras oméga-3 selon l'une quelconque des revendications 1 à 20 et 27, ledit procédé comprenant de :

a) mélanger un premier tensio-actif avec une solution saline ; et
b) mettre en contact au moins un acide oméga-6 et au moins un acide gras oméga-3 avec la solution de l'étape a).

**24.** Procédé selon la revendication 23, dans lequel ledit mélange et/ou ladite mise en contact est effectué (e) à la température ambiante.

**25.** Procédé selon la revendication 23, dans lequel ledit au moins acide gras oméga-6 et au moins un acide gras oméga-3 est mis en contact avec une solution de l'étape a) pendant une heure.

**26.** Procédé selon la revendication 23, dans lequel un second tensio-actif est mélangé avec une solution de l'étape a) avant de passer à l'étape b).

**27.** Composition selon la revendication 1, dans laquelle la quantité dudit acide gras oméga-6 est 0,005 % en poids, et dans laquelle la quantité dudit acide gras oméga-3 est 0,005 % en poids.

**Figure 1**

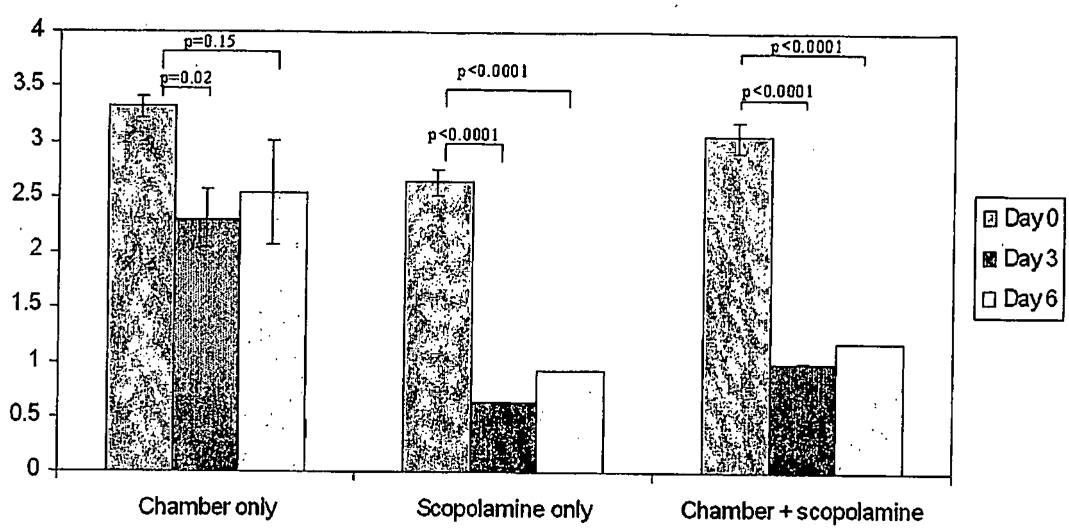
4
3.5
3
2.5
2
1.5
1
0.5
0
p=0.15
p=0.02
p<0.0001
p<0.0001
p<0.0001
p<0.0001
Day 0
Day 3
Day 6
Chamber only
Scopolamine only
Chamber + scopolamine

Figure 2

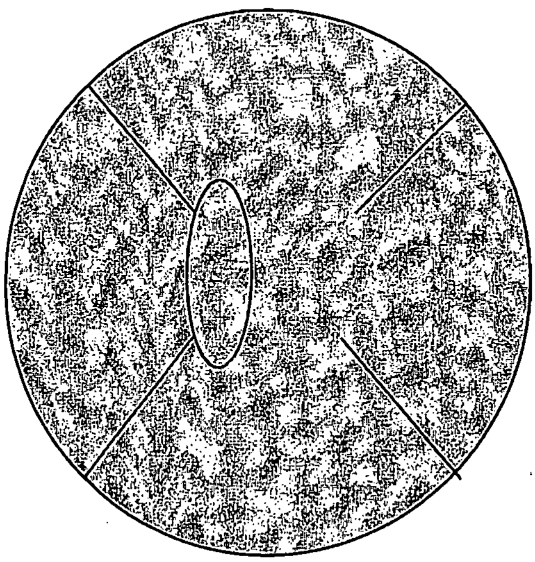

**Figure 3**

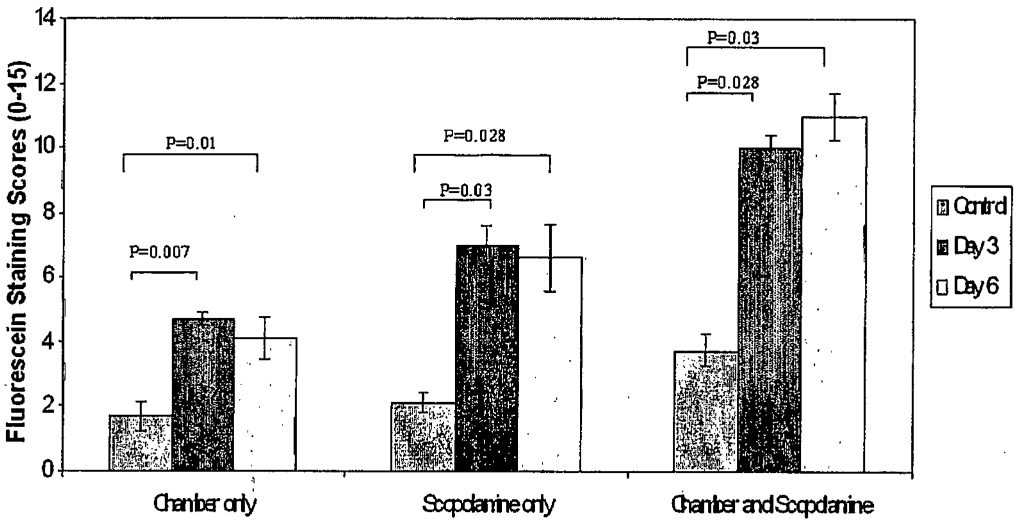
14
12
10
8
6
4
2
0
Fluorescein Staining Scores (0-15)
P=0.007
P=0.01
P=0.03
P=0.028
P=0.028
P=0.03
Control
Day3
Day6
Chamber only
Scopolamine only
Chamber and Scopolamine

**Figure 4**

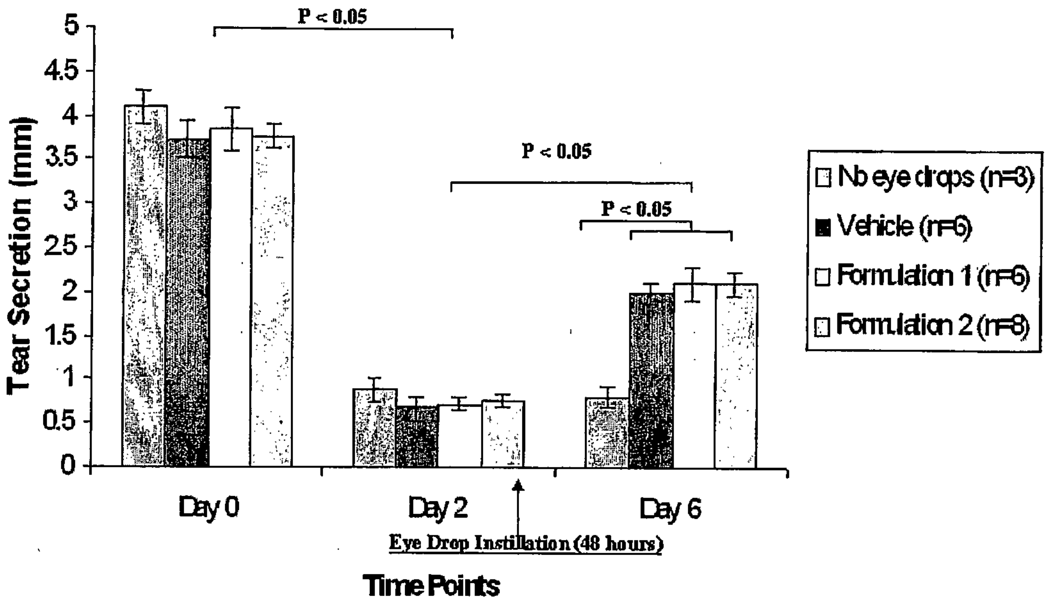
P < 0.05
P < 0.05
P < 0.05
5
4.5
4
3.5
3
2.5
2
1.5
1
0.5
0
Tear Secretion (mm)
No eye drops (n=3)
Vehicle (n=6)
Formulation 1 (n=6)
Formulation 2 (n=8)
Day 0
Day 2
Day 6
Eye Drop Instillation (48 hours)
Time Points

**Figure 5**

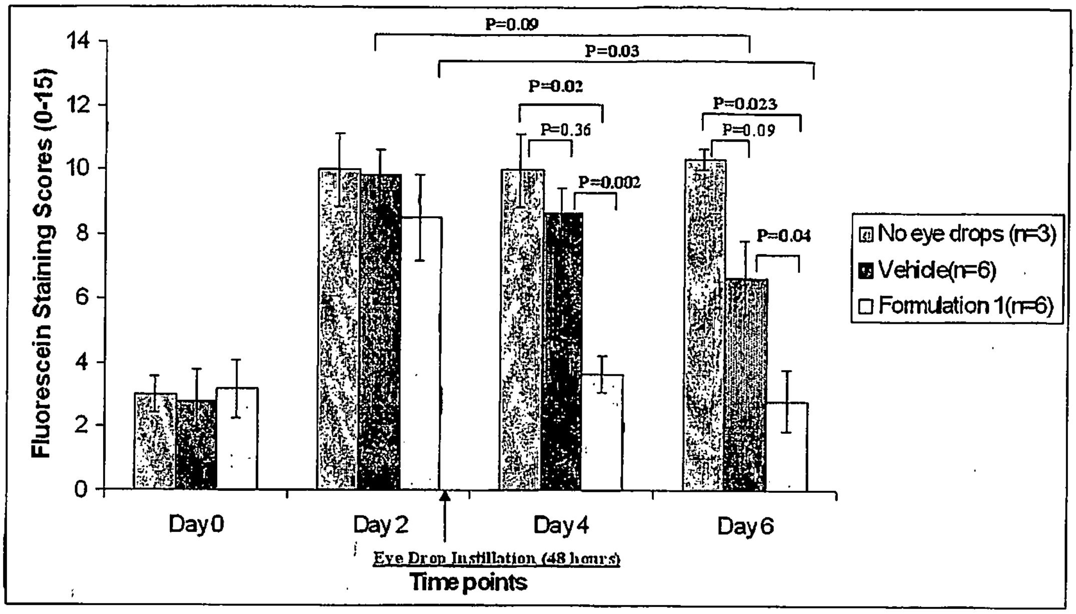
P=0.09
P=0.03
P=0.02
P=0.36
P=0.002
P=0.023
P=0.09
P=0.04
14
12
10
8
6
4
2
0
Fluorescein Staining Scores (0-15)
Day 0
Day 2
Day 4
Day 6
Eye Drop Instillation (48 hours)
Time points
No eye drops (n=3)
Vehicle(n=6)
Formulation 1(n=6)

**Figure 6**

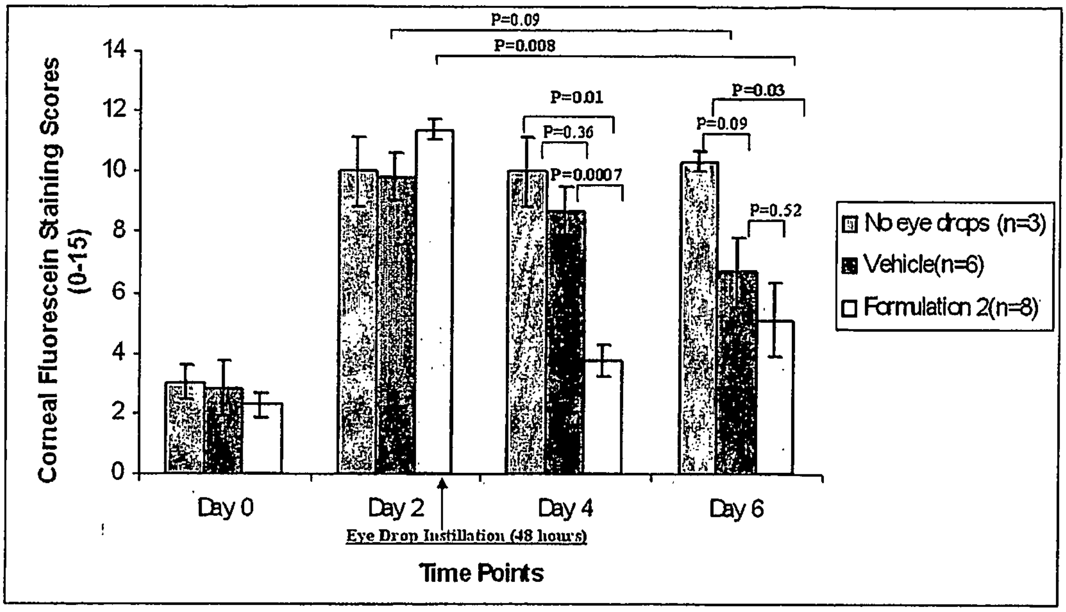
P=0.09
P=0.008
P=0.01
P=0.36
P=0.0007
P=0.03
P=0.09
P=0.52
Corneal Fluorescein Staining Scores (0-15)
14
12
10
8
6
4
2
0
No eye drops (n=3)
Vehicle(n=6)
Formulation 2(n=8)
Day 0
Day 2
Day 4
Day 6
Eye Drop Instillation (48 hours)
Time Points

**Figure 7**

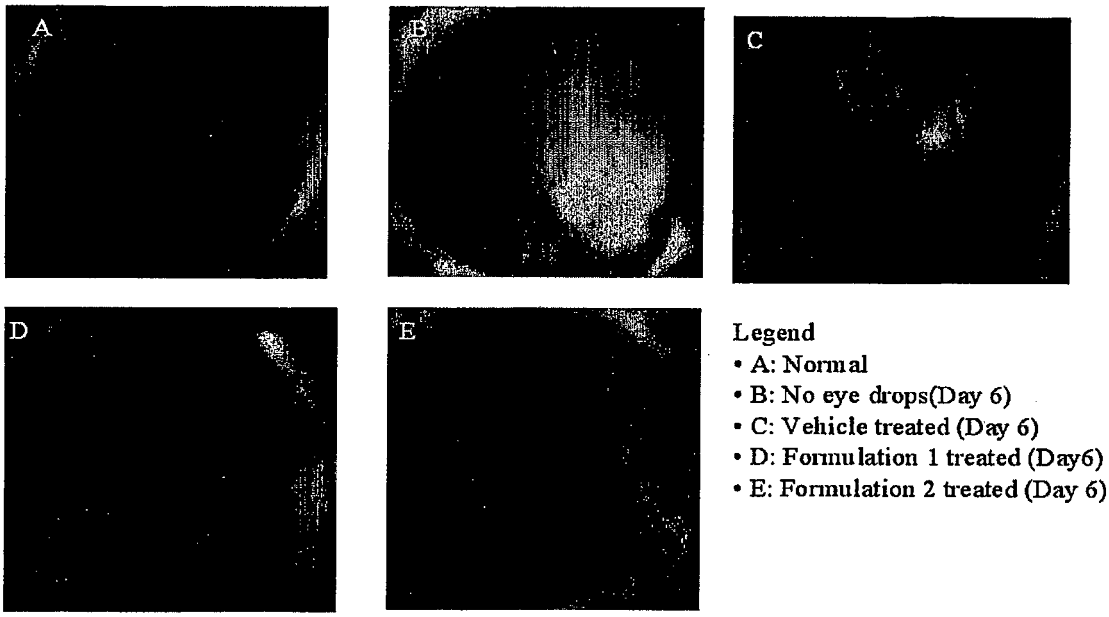


Legend
- A: Normal
- B: No eye drops(Day 6)
- C: Vehicle treated (Day 6)
- D: Formulation 1 treated (Day6)
- E: Formulation 2 treated (Day 6)

**Figure 8**

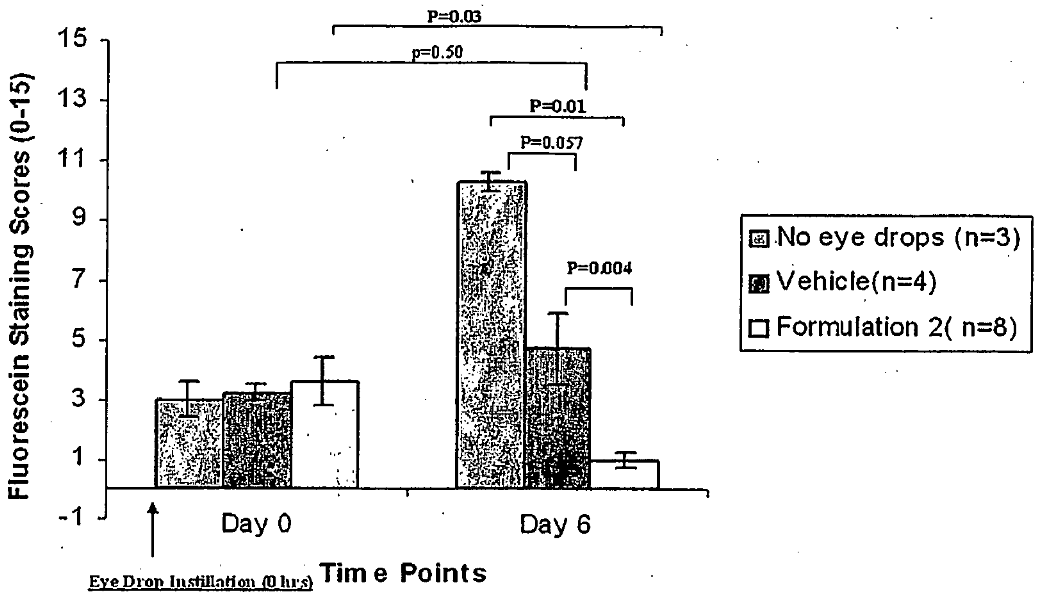

P=0.03
p=0.50
P=0.01
P=0.057
P=0.004
15
13
11
9
7
5
3
1
-1
Fluorescein Staining Scores (0-15)
Day 0
Day 6
Eye Drop Instillation (0 hrs)
Tim e Points
No eye drops (n=3)
Vehicle(n=4)
Formulation 2( n=8)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 58451404 P
- US 20040076691 A


### Non-patent literature cited in the description


- **HONG S et al.** *J Biol Chem.,* 25 April 2003, vol. 278 (17), 14677-87
- **SERHAN CN et al.** *J Exp Med.,* 21 October 2002, vol. 196 (8), 1025-37
- **MARCHESELLI VL et al.** *J Biol Chem.,* 31 October 2003, vol. 278 (44), 43807-17
- **SERHAN CN et al.** *J Immunol.,* 15 December 2003, vol. 171 (12), 6856-65
- **HAMRAH P et al.** *Arch Ophthalmol.,* 2003, vol. 121, 1132-40
- **LUO L et al.** *Invest Ophthalmol Vis Sci.,* December 2004, vol. 45 (12), 4293-301
- Vitreoretinal Surgical Techniques. Martin Dunitz, 2001
- Handbook of Pharmaceutical Controlled Release Technology. Marcel Dekker, 2000